# EUROPEAN PATENT APPLICATION

(11) **EP 3 639 873 A1**
(43) Date of publication of application: **22.04.2020**
(21) Application number: 19785281.7
(22) Date of filing: 09.04.2019
(51) Int. Cl.: A61M 5/32, A61M 5/158, A61M 37/00

(54) **MULTI-NEEDLE MODULE SUITABLE FOR SKIN PROCEDURE AT SLOPE**

(30) Priority: 13.04.2018 KR 20180043486
(71) Applicant: Bomtech Electronics Co., Ltd., Seoul 07646 (KR)
(72) Inventor: LEE, Jong-dae, Seoul 07646 (KR)
(74) Representative: Lavoix
(86) International application number: PCT/KR2019/004180
(87) International publication number: WO 2019/199009

(57) **Abstract**

A multi-needle module suitable for skin procedure at slope is disclosed. The multi-needle module includes: a housing part; and a plurality of needles configured to be disposed in parallel in the housing part, wherein tips of the needles have an arrangement along a plane Sl, and the plane Sl is a plane inclined by a predetermined angle with respect to a plane P2 perpendicular to the needles.

## Description

### [Technical Field]

The disclosure relates to a multi-needle module used to permeate a permeation target such as a nutrient or a drug into a skin, and more particularly, to a multi-needle module that may be suitably used for skin treatment in an inclined direction rather than a vertical direction.

### [Background Art]

A multi-needle module is a surgical tool used to permeate a permeation target such as a nutrient or a drug into the skin for the purpose of skin treatment, skin aging prevention, skin beauty, skin shaping, and the like, and has advantages in that a treatment time is shortened and an evenly treatment is possible because a plurality of needles operating simultaneously are regularly arranged.

The multi-needle module may be classified into a direct injection module and an indirect injection module according to the type of needle. In the specification, the direct injection module refers to a module including hollow-type needles having injection passages extending in a length direction therein, and the indirect injection module refers to a module including solid-type needles without such injection passages.

The direct injection module is used for the purpose of receiving an infusion from a syringe connected thereto and directly permeating the infusion into the skin through the hollow-type needles and the indirect injection module is used for the purpose of simply punching the skin to permeate a permeation target pre-applied to a skin surface into the skin.

The conventional multi-needle modules have a structure for permeating needles in a direction perpendicular to the skin. However, when the vertical treatment targets a shallow skin layer, a portion of a tip of the needle having an inclined-cut shape may be exposed to the outside of the skin, and a problem of leaking the injection target through the exposed portion during the treatment may occur.

In a case in which an inclined treatment is performed with the multi-needle module, an insertion length of the tip of the needle becomes relatively larger than that of the vertical treatment when the skin layer of the same depth is targeted. Therefore, the above-mentioned problem that may occur when the vertical treatment is performed for the shallow skin layer may be solved by the inclined treatment.

However, since the conventional multi-needle modules are mostly for vertical treatment, the development of the multi-needle module for inclined treatment, which may be suitably used for skin layer treatment of a shallow depth, is urgently needed.

In addition, since the inclined treatment is often preferable to the vertical treatment according to the purpose of the skin treatment, the characteristics of a target site of the treatment, the characteristics of the permeation target, there is a great need to develop a multi-needle module for inclined treatment.

### [Disclosure]

### [Technical Problem]

The disclosure has been made in view of the need of the above-mentioned multi-needle module for inclined treatment, and provides a multi-needle module for inclined treatment that may be suitably used for treatment of a skin layer of a shallow depth.

### [Technical Solution]

According to an embodiment of the disclosure, a multi-needle module includes: a housing part; and a plurality of needles configured to be disposed in parallel in the housing part, wherein tips of the needles have an arrangement along a plane Sl, and the plane Sl is a plane inclined by a predetermined angle with respect to a plane P2 perpendicular to the needles.

The multi-needle module may further include a plurality of needle guides that partially accommodate the needles, wherein tip surfaces of the needle guides may be arranged along the plane Sl.

The tip surfaces of the needle guides may be formed as a plane perpendicular to the needles.

The tip surfaces of the needle guides may be formed as a plane having the same inclination as that of the plane Sl.

The housing part may have a skin contact surface, and the skin contact surface may be disposed along the plane Sl.

The housing part may include four side portions which are parallel to the needles, and four inclined tip edge surfaces positioned at tips of the four side portions may constitute the skin contact surface.

According to another embodiment of the disclosure, a multi-needle module includes: a housing part; and a plurality of needles configured to be disposed in parallel in the housing part, wherein tips of the needles have an arrangement along a curved surface S2, the curved surface S2 is a curved surface disposed to be inclined with respect to a plane P2 perpendicular to the needles, when viewed in at least one direction perpendicular to the needles.

The curved surface S may be a curved surface disposed to be inclined with respect to the plane P2 perpendicular to the needles, when viewed in only one direction perpendicular to the needles.

The curved surface S may be a curved surface disposed to be inclined with respect to the plane P2 perpendicular to the needles, when viewed in two directions perpendicular to the needles, and the two directions may be directions perpendicular to each other.

The multi-needle module may further include a plurality of needle guides that partially accommodate the needles, wherein tip surfaces of the needle guides may be arranged along the curved surface Sl.

The tip surfaces of the needle guides may be formed as a plane perpendicular to the needles.

The tip surfaces of the needle guides may be formed as a curved surface forming a portion of the curved surface S2.

The housing part may have a skin contact surface, and the skin contact surface may be disposed along the curved surface S2.

The housing part may include four side portions which are parallel to the needles, and four inclined tip edge surfaces positioned at tips of the four side portions may constitute the skin contact surface.

### [Advantageous Effects]

Since the multi-needle module according to the disclosure has the skin contact surface disposed to be inclined with respect to the needle at the front end of the housing part, the multi-needle module may be suitably used for skin treatment in the inclined direction.

In the case in which the syringe is connected to the multi-needle module according to the disclosure to inject the nutrient or the drug into the skin, even if the skin permeation depth of the needle is set to be shallow, the tip of the needle may permeate into the skin sufficiently as compared to a general module, thereby preventing loss of the nutrient or the drug through the tip portion of the needle exposed to the outside of the skin. Therefore, the multi-needle module according to the disclosure may be usefully used to inject the nutrient or the drug into the skin layer of the shallow depth.

### [Description of Drawings]

FIG. 1 is a perspective view illustrating a multi-needle module according to an embodiment of the disclosure.
FIG. 2 is a side view of the multi-needle module of FIG. 1.
FIGS. 3 and 4 are partial cross-sectional views taken a line A-A' of the multi-needle module of FIG. 1, where FIG. 3 illustrates an initial state and FIG. 4 illustrates needles coming out of the module during treatment.
FIGS. 5 and 6 are views for comparing the multi-needle module of FIG. 1 with the conventional multi-needle module, where FIG. 5 illustrates a usage example of the conventional multi-needle module and FIG. 6 illustrates a usage example of the multi-needle module of FIG. 1.
FIG. 7 is a side view of a multi-needle module according to a second embodiment of the disclosure.
FIG. 8 is a partial cross-sectional view of the multi-needle module of FIG. 7.
FIG. 9 is a partial cross-sectional view of the multi-needle module of FIG. 7 and illustrates another cross-section perpendicular to a cross section of FIG. 8.
FIG. 10 is a view illustrating another example of a needle guide of the multi-needle module of FIG. 7.

### [Best Mode]

Hereinafter, a multi-needle module for inclined treatment according to an embodiment of the disclosure will be described in detail with reference to the accompanying drawings.

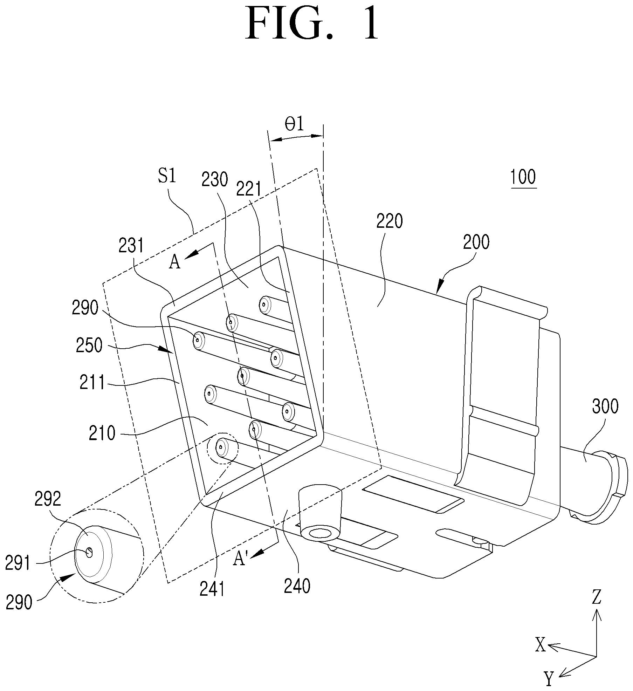

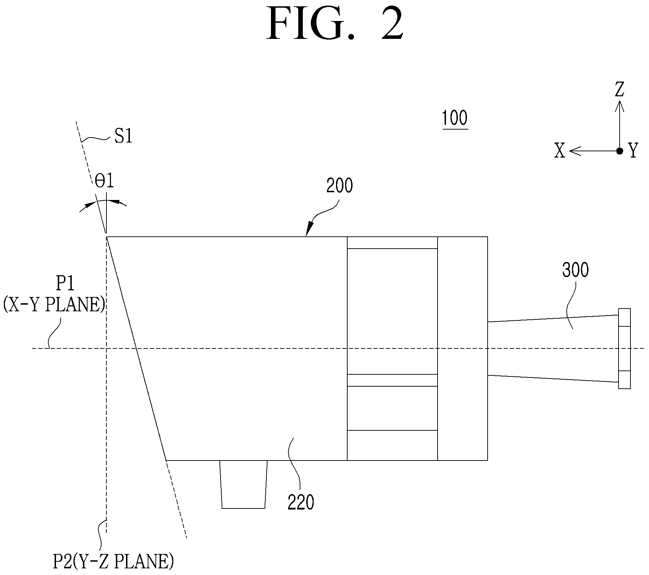

FIG. 1 is a perspective view illustrating a multi-needle module 100 for inclined treatment according to an embodiment of the disclosure, FIG. 2 is a side view of the multi-needle module 100 of FIG. 1, and FIGS. 3 and 4 are partial cross-sectional views taken a line A-A' of the multi-needle module 100 of FIG. 1.

Referring to FIGS. 1 through 4, a multi-needle module 100 for inclined treatment includes a housing part 200, a connection port 300, a needle mounting part 400, and a plurality of needles 500.

The housing part 200 is a configuration forming a frame of the multi-needle module 100 and has a box shape of a substantially hexahedron shape that is opened in the front and the rear.

The housing part 200 includes a vertical wall 270 dividing an internal space of the housing part 200 into two, and a plurality of needle guides 290 vertically protruding from the vertical wall 270 toward a skin contact surface 250.

The needle guide 290 is a configuration for guiding a movement of the needle 500 during treatment, has a hollow formed therein to accommodate a portion of the needle 500, and has a needle entrance hole 291 connected to the hollow formed therein and formed in a tip surface 292 (see FIG. 1) thereof.

The connection port 300 is a configuration connected to a syringe or an operation aid. In a case in which the multi-needle module 100 is a direct injection module, the multi-needle module 100 is used by connecting a syringe accommodating an infusion to the connection port 300, and in a case in which the multi-needle module 100 is an indirect injection module, the multi-needle module 100 is used by connecting an operation aid for aiding only a needle permeation operation of the multi-needle module 100 to the connection port 300.

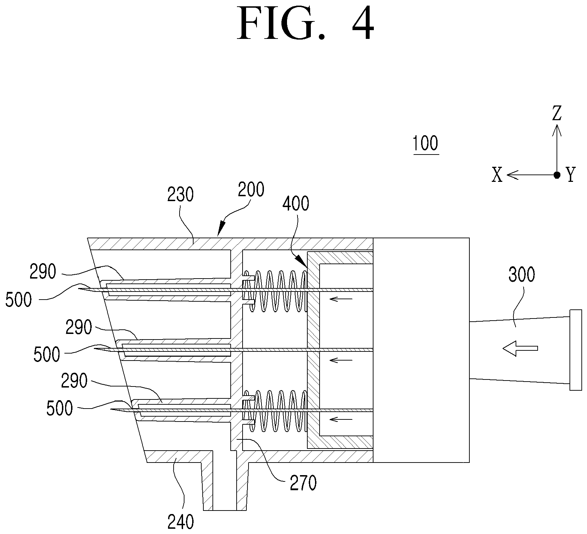

The needle mounting part 400 is configuration on which the plurality of needles 500 are mounted and one side thereof is connected to the connection port 300. As illustrated in FIG. 4, when the syringe or the operation aid connected to the connection port 300 is driven forward, the needle mounting part 400 connected to the connection port 300 also moves forward, and as a result, the needles 500 stick out of the needle guides 290 and permeate into a skin.

In the case in which the multi-needle module 100 is the direct injection module, an infusion distribution chamber (not illustrated) is formed in the needle mounting part 400. Such a distribution chamber serves to receive the infusion from the syringe through the connection portion 300 and uniformly distribute the infusion to each of the needles.

The plurality of needles 500 may be provided as hollow-type needles or solid-type needles. In the case in which the multi-needle module is the direct injection module used in connection with the syringe, the plurality of needles 500 are provided as the hollow-type needles in which an injection passage is formed, and in the case in which the multi-needle module 100 is the indirect injection module used in connection with the operation aid, the plurality of needles 500 are provided as the solid-type needles without the injection passage therein.

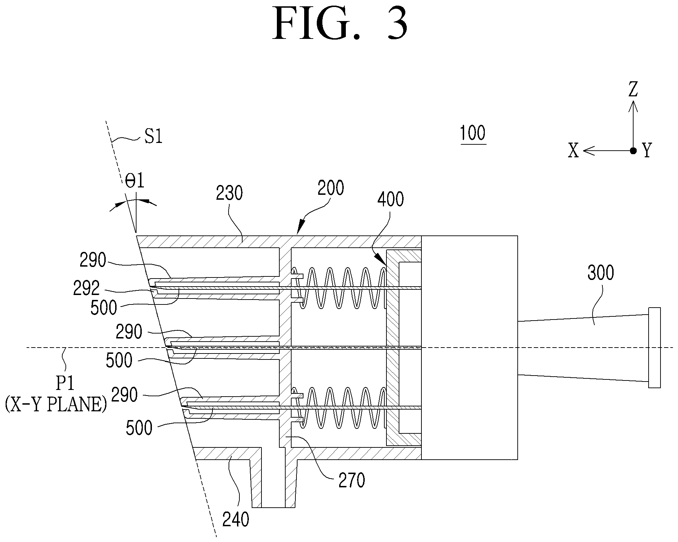

Referring to FIGS. 2 and 3, tips of the needles 500 have an arrangement along a plane Sl and the plane Sl is a plane inclined at a predetermined angle θ1 with respect to a plane P2 perpendicular to the needles 500. Here, the plane P2 refers to a Y-Z plane based on coordinates indicated in FIG. 2.

The tips of the needles 500 have the inclined arrangement along the plane Sl, thereby making it possible to suitably use the multi-needle module 100 according to the embodiment for the inclined treatment.

The plurality of needles 500 have a length that is gradually longer as the needles are in an upward direction. More specifically, the multi-needle module 100 according to the embodiment has a total of nine needles 500, where three lower needles 500 adjacent to a third side portion 240 have the shortest length, three upper needles 500 adjacent to a fourth side portion 230 have the longest length, and the remaining three middle needles 290 disposed in the middle have a medium length.

In the embodiment, tip surfaces 292 of the needle guides 290 are also arranged along the plane Sl. The tip surfaces 292 of the needle guides 290 together with the needles 500 have the arrangement along the plane Sl, thereby making it possible to more suitably use the multi-needle module 100 according to the embodiment for the inclined treatment.

As illustrated in FIG. 3, the tip surfaces 292 of the needle guides 290 may be formed as a plane having the same inclination as that of the plane Sl. Alternatively, the tip surfaces 292 may also be formed as a plane perpendicular to the needles.

As illustrated in FIG. 3, like the needles 500, the needle guides 290 have a length that is gradually longer as the needle guides are in an upward direction. More specifically, the multi-needle module 100 according to the embodiment has a total of nine needle guides 290, where three lower needle guides 290 adjacent to a third side portion 240 have the shortest length, three upper needle guides 500 adjacent to a fourth side portion 230 have the longest length, and the remaining three middle needle guides 290 disposed in the middle have a medium length.

The housing part 200 may have four side portions 210, 220, 230, and 240. The first and second side portions 210 and 220 disposed on the left and right sides are disposed to oppose each other in parallel, and the third and fourth side portions 230 and 240 disposed on the upper and lower sides are disposed to oppose each other in parallel. In addition, the four side portions 210, 220, 230, 240 are disposed in parallel to the needles 500.

As illustrated in FIG. 2, the first and second side portions 210 and 220 disposed on the left and right sides have a trapezoidal shape in which only one side edge is inclined, whereas the third and fourth side portions 230 and 240 disposed on the upper and lower sides have a rectangular shape without the inclined edge.

The respective side portions 210, 220, 230, and 240 have tip edge surfaces 211, 221, 231, and 241. Such four tip edge surfaces 211, 221, 231, and 241 are connected to each other to form one skin contact surface 250. The skin contact surface 250 is in close contact with a target skin during the treatment using the multi-needle module 100. Such a skin contact surface 250 is disposed along the plane Sl.

In the embodiment, the skin contact surface 250 has a square shape, but the shape of the skin contact surface 250 may vary according to the shape of the housing part 200. For example, when the housing part 200 is manufactured in a cylindrical shape, the skin contact surface 250 becomes an ellipse shape.

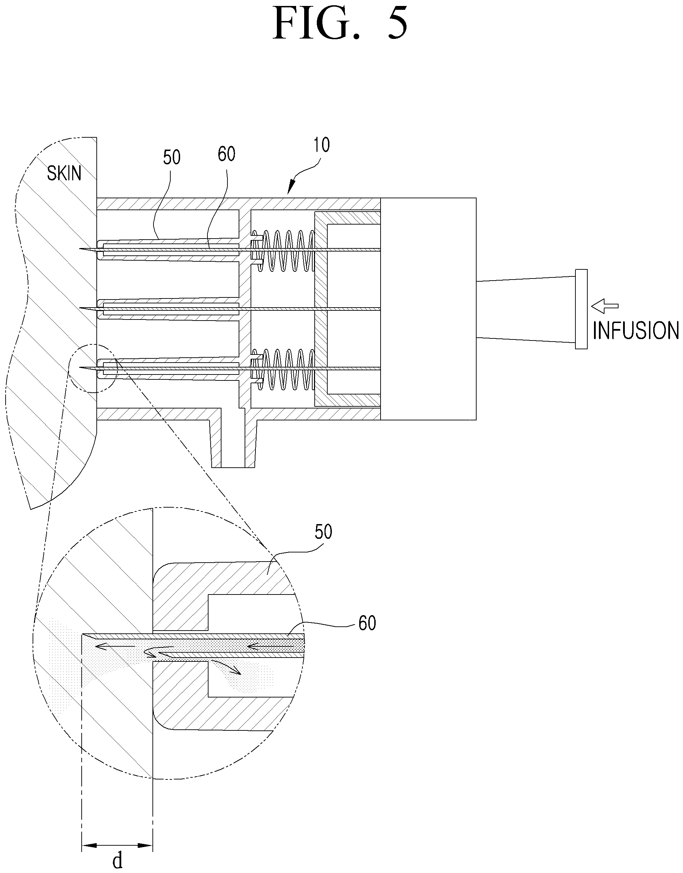

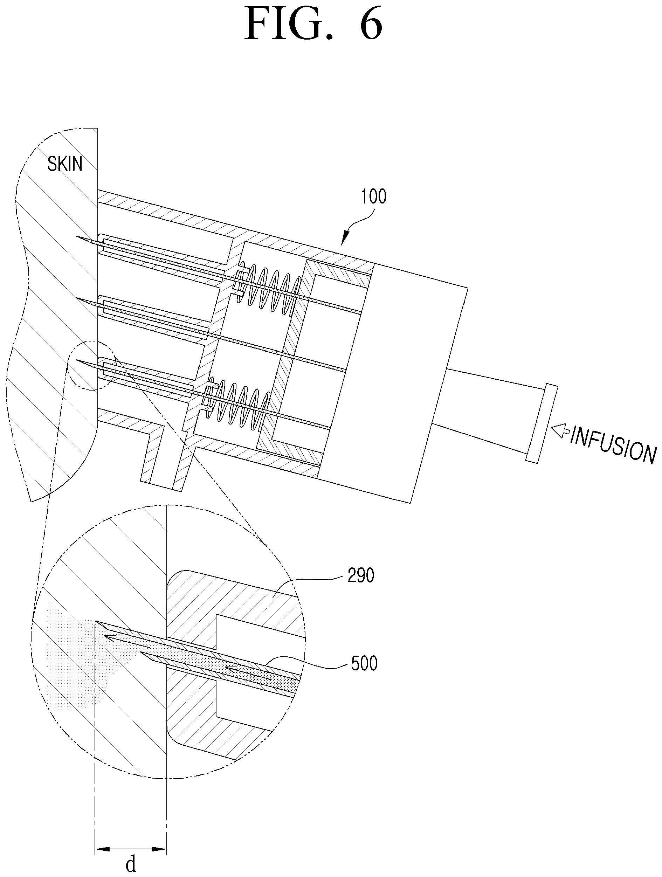

FIGS. 5 and 6 are views for comparing the multi-needle module of FIG. 1 with a general multi-needle module, where FIG. 5 illustrates a usage example of the conventional multi-needle module and FIG. 6 illustrates a usage example of the multi-needle module of FIG. 1.

First, referring to FIG. 5, when the nutrient or drug is injected into the skin at a shallow depth d with a general multi-needle module 10 having an arrangement in which a skin contact surface 60 is perpendicular to the needle 60, a portion of the tip of the needle having an inclined-cut form may not perfectly permeate into the skin and the portion may be exposed to the outside of the skin, and as a result, some of the injection target may be leaked to the outside through the exposed tip of the needle and wasted.

Next, referring to FIG. 6, when the injection target is injected into a skin layer of the same shallow depth d with the multi-needle module according to the embodiment of the disclosure, a portion of the tip of the needle of the inclined-cut form may not be exposed to the outside and the entirety of the tip of the needle may permeate into the skin, thereby preventing the injection target from being wasted by the exposed tip of the needle.

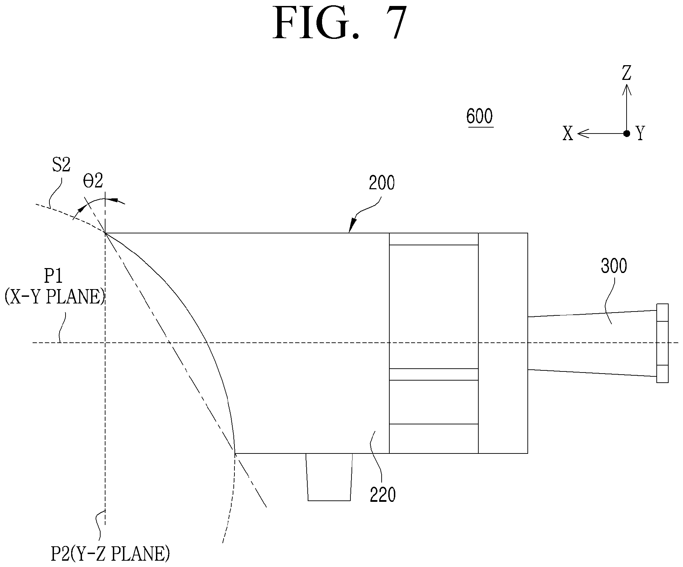

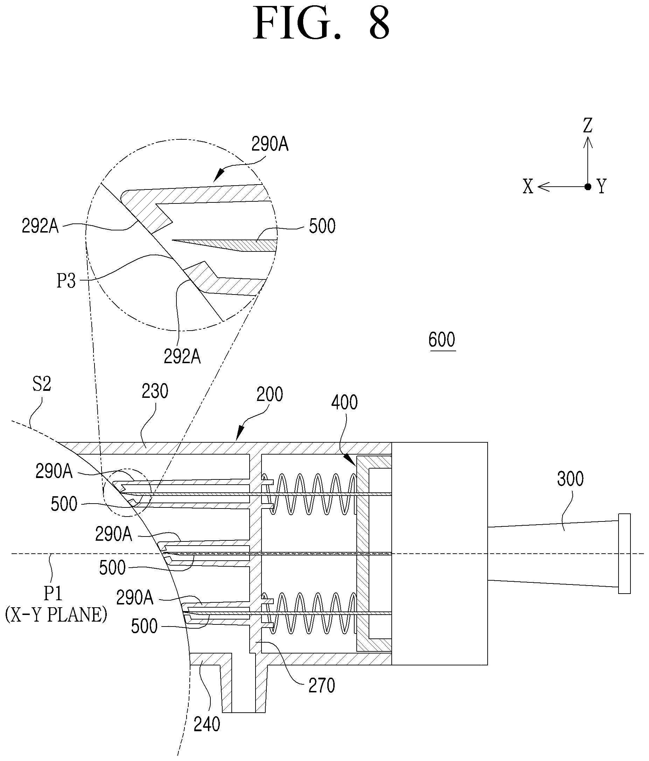

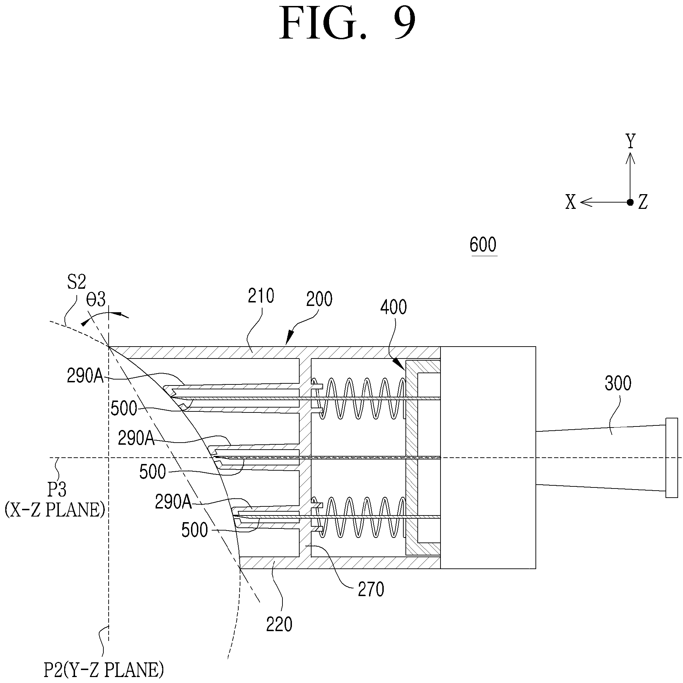

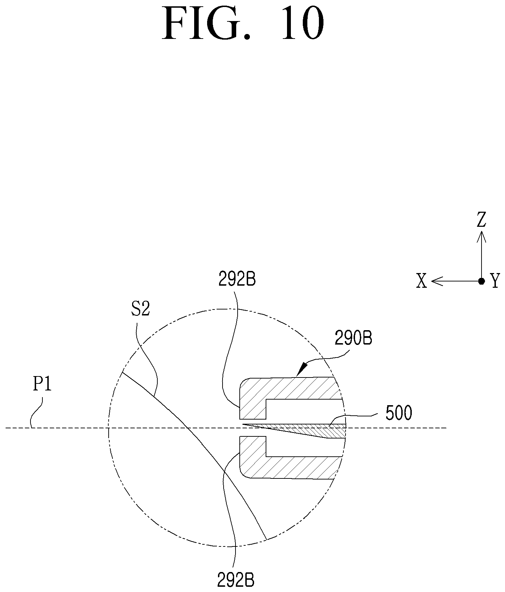

FIG. 7 is a side view of a multi-needle module 600 according to a second embodiment of the disclosure, FIG. 8 is a partial cross-sectional view of the multi-needle module 600 of FIG. 7, FIG. 9 is a partial cross-sectional view of the multi-needle module of FIG. 7 and illustrates another cross-section perpendicular to a cross section of FIG. 8, and FIG. 10 is a view illustrating another example of a needle guide 290A.

Compared with the multi-needle module 100 according to the first embodiment described above, a difference from the multi-needle module 600 according to the second embodiment will be mainly described.

The tips of the needles 500 of the multi-needle module 600 according to the second embodiment have an arrangement along a curved surface S2, unlike the multi-needle module 600 according to the first embodiment described above.

In the disclosure, the curved surface S2 is a curved surface disposed to be inclined with respect to the plane P2 perpendicular to the needles 500 when viewed in at least one direction perpendicular to the needles 500.

The embodiment illustrates a case in which the curved surface S2 is a curved surface disposed to be inclined with respect to the plane P2 when viewed in two directions perpendicular to the needles 500. Specifically, the curved surface S2 is a curved surface inclined by a predetermined angle θ2 with respect to the plane P2 when viewed in a first direction (Y direction in the drawing) as illustrated in FIGS. 7 and 8, and the curved surface S2 is also a curved surface inclined by a predetermined angle θ3 with respect to the plane P2 when viewed in a second direction (Z direction in the drawing) perpendicular to the first direction as illustrated in FIG. 9.

In addition, alternatively, the curved surface S2 may also be a curved surface disposed to be inclined with respect to the plane P2 only when viewed in only one direction perpendicular to the needles 500.

The tips of the needles 500 are arranged along the curved surface Sl disposed to be inclined as described above, thereby making it possible to suitably use the multi-needle module 100 according to the embodiment for the inclined treatment.

The multi-needle module 600 according to the second embodiment includes a plurality of needle guides 290A and tip surfaces 292A of the needle guides 290A are arranged along the curved surface S2 described above. As such, the tip surfaces 292A of the needle guides 290A are arranged along the curved surface S2 described above, thereby making it possible to more suitably use the multi-needle module 600 for the inclined treatment.

As illustrated in FIG. 8, the tip surfaces 292B of the needle guides 290 may form a portion of the curved surface S2 as a curved surface. Alternatively, as illustrated in FIG. 10, the tip surfaces 292B may also be formed as a plane perpendicular to the needles 500.

The multi-needle module 600 includes a housing part 200, and the skin contact surface disposed at the tip of the housing part 200 may also be preferably disposed along the curved surface S2 similarly to the tip surfaces 292B described above.

Although the embodiments of the disclosure have been described hereinabove, it will be appreciated that those skilled in the art may variously modify and change the disclosure within the scope without departing from the spirit and scope of the disclosure described in the claims below.

### [Industrial Applicability]

The disclosure relates to a multi-needle module for inclined treatment that may be used particularly to be suitable for the treatment for the skin layer of shallow depth.

## Claims

1. A multi-needle module for skin treatment in an inclined direction, the multi-needle module comprising:
a housing part; and
a plurality of needles configured to be disposed in parallel in the housing part,
wherein tips of the needles have an arrangement along a plane Sl, and
the plane Sl is a plane inclined by a predetermined angle with respect to a plane P2 perpendicular to the needles.

2. The multi-needle module as claimed in claim 1, further comprising a plurality of needle guides,
wherein tip surfaces of the needle guides are arranged along the plane Sl.

3. The multi-needle module as claimed in claim 2, wherein the tip surfaces of the needle guides are formed as a plane perpendicular to the needles.

4. The multi-needle module as claimed in claim 2, wherein the tip surfaces of the needle guides are formed as a plane having the same inclination as that of the plane Sl.

5. The multi-needle module as claimed in claim 1, wherein the housing part has a skin contact surface, and
the skin contact surface is disposed along the plane S1.

6. The multi-needle module as claimed in claim 5, wherein the housing part includes four side portions which are parallel to the needles, and
four inclined tip edge surfaces positioned at tips of the four side portions constitute the skin contact surface.

7. A multi-needle module for skin treatment in an inclined direction, the multi-needle module comprising:
a housing part; and
a plurality of needles configured to be disposed in parallel in the housing part,
wherein tips of the needles have an arrangement along a curved surface S2, and
the curved surface S2 is a curved surface disposed to be inclined with respect to a plane P2 perpendicular to the needles, when viewed in at least one direction perpendicular to the needles.

8. The multi-needle module as claimed in claim 7, wherein the curved surface S is a curved surface disposed to be inclined with respect to the plane P2 perpendicular to the needles, when viewed in only one direction perpendicular to the needles.

9. The multi-needle module as claimed in claim 7, wherein the curved surface S is a curved surface disposed to be inclined with respect to the plane P2 perpendicular to the needles, when viewed in two directions perpendicular to the needles, and
the two directions are directions perpendicular to each other.

10. The multi-needle module as claimed in claim 7, further comprising a plurality of needle guides,
wherein tip surfaces of the needle guides are arranged along the curved surface Sl.

11. The multi-needle module as claimed in claim 10, wherein the tip surfaces of the needle guides are formed as a plane perpendicular to the needles.

12. The multi-needle module as claimed in claim 10, wherein the tip surfaces of the needle guides are formed as a curved surface forming a portion of the curved surface S2.

13. The multi-needle module as claimed in claim 7, wherein the housing part has a skin contact surface, and
the skin contact surface is disposed along the curved surface S2.

14. The multi-needle module as claimed in claim 13, wherein the housing part includes four side portions which are parallel to the needles, and
four inclined tip edge surfaces positioned at tips of the four side portions constitute the skin contact surface.
